# EUROPEAN PATENT APPLICATION

(11) **EP 2 910 172 A1**
(43) Date of publication of application: **26.08.2015**
(21) Application number: 13846413.6
(22) Date of filing: 10.10.2013
(51) Int. Cl.: A61B 1/00, A61B 1/04

(54) **IMAGE PROCESSING DEVICE AND IMAGE PROCESSING METHOD**

(30) Priority: 18.10.2012 JP 2012231183
(71) Applicant: Olympus Medical Systems Corp., Tokyo 151-0072 (JP)
(72) Inventor: KOBAYASHI, Ryota, Tokyo 151-0072 (JP); TANIGUCHI, Katsuyoshi, Tokyo 151-0072 (JP)
(74) Representative: Gunzelmann, Rainer
(86) International application number: PCT/JP2013/077613
(87) International publication number: WO 2014/061554

(57) **Abstract**

An image processing apparatus that can reduce a time for the user to perform necessary determinations after completion of an examination, as compared with a conventional technology, is provided. In a capsule endoscopic system including a capsule endoscope introduced into a subject and wirelessly transmitting a series of image data generated by capturing images inside the subject, and a receiver receiving and accumulating the series of the image data wirelessly transmitted from the capsule endoscope, the image processing apparatus 5 that processes the image data acquired from the receiving device includes an image data acquisition unit 52 that sequentially acquires the image data from the receiving device in order from a latest imaging time, an image processing unit 54 that performs predetermined image processing on the image data acquired by the image data acquisition unit 52, in order in which the image data are acquired, and a display controller 55 that displays a screen containing a result obtained through the predetermined image processing.

## Description

### Field

The present invention relates to an image processing apparatus and an image processing method, which process images acquired by a capsule endoscope in examinations using the capsule endoscope that is introduced into a subject and captures images of inside of the subject. Background

In recent years, examinations using capsule endoscopes (hereinafter, referred to as a capsule endoscopic examination or simply as an examination) which are introduced into subjects such as patients and capture images of insides of the subjects are known in the field of endoscopes. A capsule endoscope is an apparatus that has a built-in imaging function, a built-in wireless communication function, and the like provided in a casing of a capsule shape formed in a size introducible into a digestive tract of a subject.

In general, a capsule endoscopic examination is performed as described below. First, a medical worker such as a nurse attaches an antenna unit on the outside surface of a body of a patient that is a subject, and connects, to the antenna unit, a receiving device enabled to perform wireless communications with the capsule endoscope. Then, an imaging function of the capsule endoscope is turned on and the capsule endoscope is swallowed by the patient. Accordingly, the capsule endoscope is introduced into the subject, captures images while moving inside the digestive tract by peristaltic movement or the like, and wirelessly transmits image data of in-vivo images. The image data are received by the receiving device and accumulated in a built-in memory. Thereafter, the patient is allowed to freely act, for example, go out from the hospital, until the time designated by the medical worker as long as the patient carries the receiving device.

When the patient comes back to the hospital at the designated time, the examination is temporarily suspended, and the medical worker removes the receiving device from the patient and connects the receiving device to an image processing apparatus that is configured with a workstation or the like. Therefore, the image data accumulated in the receiving device are downloaded (transferred) to the image processing apparatus, and the image processing apparatus performs predetermined image processing to form images. The medical worker observes in-vivo images displayed on a screen of the image processing apparatus, confirms that the capsule endoscope has reached a large intestine, has captured a necessary region inside the subject, and has generated image data without a communication failure (a failure of an antenna) or a shortage of a battery after being swallowed, and then allows the patient to go home. Thereafter, the medical worker clears away devices, such as the receiving device, and finishes his/her work.

As a technique related to confirmation of the end of a capsule endoscopic examination, Patent Literature 1 discloses a technique for analyzing the last images or a plurality of images received from an imaging apparatus, and determining whether the imaging apparatus is located inside a living body.

### Citation List

### Patent Literature

Patent Literature 1: Japanese Laid-open Patent Publication No. 2009-297497

### Summary

### Technical Problem

As described in the above, when the capsule endoscopic examination is performed, a nurse has to wait until completion of the downloading of the image data and the image processing to confirm, on a screen, that the capsule endoscope has reached the large intestine and the capturing of the images in the subject has been completed normally. Moreover, a doctor performing a diagnosis also has to wait the completion of the downloading of the image data and the image processing to grasp a range (organs) that the capsule endoscope has observed and confirm whether or not in-vivo images necessary for the diagnosis have been acquire. Furthermore, if the capturing has not finished normally and the necessary images have not been acquired, re-examination is required and so a patient has to wait.

However, because a series of images captured by the capsule endoscope counts, for example, sixty thousands, the downloading and the image processing of the image data takes a long time. Therefore, conventionally the medical workers and the patient had to wait for a long time after the examination until a user (the medical worker) can perform necessary determinations.

The present invention has been made considering the above and an object thereof is to provide an image processing apparatus and an image processing method that can reduce a time for the user to perform necessary determinations after completion of an examination, as compared with a conventional technology, is provided.

### Solution to Problem

To solve the above-described problem and achieve the object, an image processing apparatus according to the present invention, in a capsule endoscopic system including a capsule endoscope introduced into a subject and wirelessly transmitting a series of image data generated by capturing images inside the subject, and a receiver receiving and accumulating the series of the image data wirelessly transmitted from the capsule endoscope, processes the image data acquired from the receiving device and includes: an image data acquisition unit that sequentially acquires the image data from the receiving device in order from a latest imaging time; an image processing unit that performs predetermined image processing on the image data acquired by the image data acquisition unit, in order in which the image data are acquired; and a display controller that displays a screen containing a result obtained through the predetermined image processing.

In the above-described image processing apparatus, the image processing unit generates images based on the image data, and the display controller displays the images generated by the image processing unit in order in which the images are generated.

In the above-described image processing apparatus, the image data acquisition unit acquires image data generated in a predetermined period of time in reverse chronological order from the latest imaging time of the capsule endoscope.

In the above-described image processing apparatus, the image processing unit generates images based on the image data, and determines whether or not a specific region inside the subject appears in the images, the display controller displays a result of determination performed by the image processing unit.

In the above-described image processing apparatus, the image processing unit determines whether an organ inside the subject appears in the images based on the image data, and subsequently determines, with respect to images in which the organ appears, whether or not the organ in the images is the specific region.

In the above-described image processing apparatus, the image data acquisition unit divides the series of image data captured in one examination by the capsule endoscope into a plurality of blocks, and acquires image data in order from a latest imaging time of each of the blocks, the image processing unit sequentially generates images based on the image data, and the display controller displays a screen containing a plurality of image display areas for displaying a plurality of images based on the image data acquired from the respective blocks.

In the above-described image processing apparatus, the image data acquisition unit acquires a part of the image data from each of the blocks while sequentially changing the group from which the image data is acquired among the blocks.

In the above-described image processing apparatus, the image data acquisition unit acquires the image data from the plurality of the blocks in parallel.

An image processing method according to the present invention, in a capsule endoscopic system including a capsule endoscope introduced into a subject and wirelessly transmitting a series of image data generated by capturing images inside the subject, and a receiver receiving and accumulating the series of the image data wirelessly transmitted from the capsule endoscope, processes the image data acquired from the receiving device and includes: an image data acquiring step of acquiring the image data sequentially from the receiving device in order from a latest imaging time; an image processing step of performing predetermined image processing on the image data acquired at the acquiring, in order in which the image data are acquired; and a display controlling step of displaying a screen containing a result obtained through the predetermined image processing.

### Advantageous Effects of Invention

According to the present invention, image data accumulated in the receiving device are acquired in order from the latest imaging time, image processing is performed in the order in which the image data are acquired, and results of the image processing are displayed on a screen. Therefore, it becomes possible to reduce a time for the user to perform necessary determinations, as compared with a conventional technology.

### Brief Description of Drawings

FIG. 1 is a schematic diagram illustrating a schematic configuration of a capsule endoscopic system including an image processing apparatus according to a first embodiment of the present invention.
FIG. 2 is a diagram illustrating internal configurations of a capsule endoscope and a receiving device illustrated in FIG. 1.
FIG. 3 is a block diagram illustrating a schematic configuration of the image processing apparatus illustrated in FIG. 1.
FIG. 4 is a flowchart illustrating operations of the capsule endoscopic system illustrated in FIG. 1.
FIG. 5 is a schematic diagram for explaining operations of the image processing apparatus illustrated in FIG. 3.
FIG. 6 is a schematic diagram illustrating a display example of a screen displayed on a display device during acquisition of image data.
FIG. 7 is a schematic diagram for explaining operations of the image processing apparatus according to a first modified example.
FIG. 8 is a flowchart illustrating operations of a capsule endoscopic system including an image processing apparatus according to a second embodiment of the present invention.
FIG. 9 is a schematic diagram illustrating an example of a notification screen indicating that a large intestine is confirmed.
FIG. 10 is a schematic diagram illustrating an example of a notification screen indicating that a large intestine is not confirmed.
FIG. 11 is a schematic diagram illustrating an example of an input screen for inputting an instruction on whether to continue image processing.
FIG. 12 is a schematic diagram for explaining operations of an image processing apparatus according to a third embodiment of the present invention.
FIG. 13 is a schematic diagram illustrating an example of a screen displayed on a display unit during acquisition of image data.

### Description of Embodiments

Exemplary embodiments of an image processing apparatus and an image processing method according to the present invention will be described below with reference to the drawings. The present invention is not limited by the embodiments below. Furthermore, in describing the drawings, the same components are denoted by the same reference signs.

### (First Embodiment)

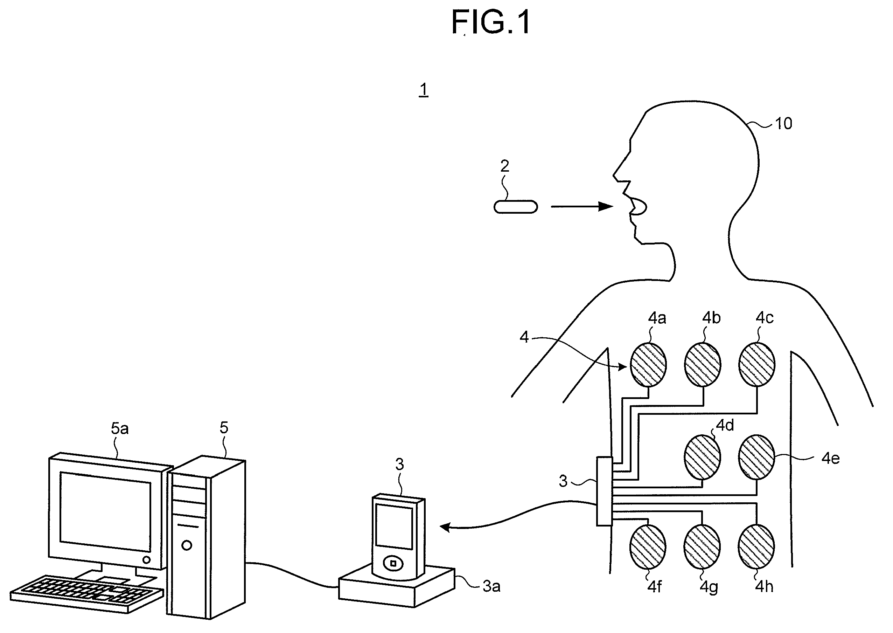

FIG. 1 is a schematic diagram illustrating a schematic configuration of a capsule endoscopic system including an image processing apparatus according to a first embodiment of the present invention. A capsule endoscopic system 1 illustrated in FIG. 1 includes: a capsule endoscope 2 that is introduced into a subject 10, that captures an image of inside of the subject 10 to generate image data, and that transmits the image data by superimposing the image data on a wireless signal; a receiving device 3 that receives the wireless signal transmitted from the capsule endoscope 2 via a receiving antenna unit 4 attached to the subject 10; and an image processing apparatus 5 that acquires the image data generated by the capsule endoscope 2 from the receiving device 3 and that performs predetermined image processing.

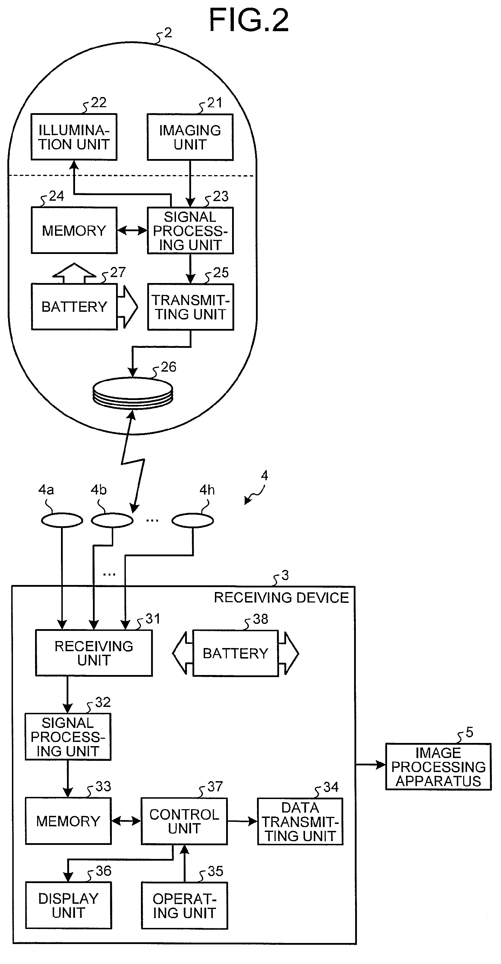

FIG. 2 is a block diagram illustrating internal configurations of the capsule endoscope 2 and the receiving device 3. The capsule endoscope 2 is a device that has various built-in parts, such as an imaging element, in a capsule shaped casing of a size swallowable by the subject 10, and includes, as illustrated in FIG. 2, an imaging unit 21 that captures an image of the inside of the subject 10; an illumination unit 22 that illuminates the inside of the subject 10 when an image is captured, a signal processing unit 23; a memory 24; a transmitting unit 25 and an antenna 26; and a battery 27.

The imaging unit 21 includes, for example: an imaging element, such as a CCD or a CMOS, that generates image data of an image representing the inside of the subject 10 based on an optical image formed on a light receiving surface; and an optical system, such as an objective lens, that is arranged on a light receiving surface side of the imaging element.

The illumination unit 22 is realized by a semiconductor light-emitting element (for example, a light emitting diode (LED)) or the like that emits light toward the inside of the subject 10 when an image is captured. The capsule endoscope 2 has a built-in circuit board (not illustrated) in which a driving circuit or the like that drives each of the imaging unit 21 and the illumination unit 22 is formed. The imaging unit 21 and the illumination unit 22 are fixed on the circuit board such that respective fields of view are directed outward from one end portion of the capsule endoscope 2.

The signal processing unit 23 controls each unit in the capsule endoscope 2, performs A/D conversion on an imaging signal output from the imaging unit 21 to generate digital image data, and further performs predetermined signal processing on the digital image data.

The memory 24 temporarily stores therein various operations executed by the signal processing unit 23 and the image data subjected to the signal processing in the signal processing unit 23.

The transmitting unit 25 and the antenna 26 superimpose, together with related information, the image data stored in the memory 24 on a wireless signal and transmits the superimposed signal to outside.

The battery 27 supplies electric power to each unit in the capsule endoscope 2. The battery 27 includes a power supply circuit that performs boosting or the like of electric power supplied from a primary battery or secondary battery, such as a button battery.

After being swallowed by the subject 10, the capsule endoscope 2 sequentially captures images of living body sites (an esophagus, a stomach, a small intestine, a large intestine, and the like) at predetermined time intervals (for example, 0.5 second time interval) while moving inside the digestive tract of the subject 10 by peristaltic movement or the like of organs. The image data and related information generated from acquired imaging signals are sequentially and wirelessly transmitted to the receiving device 3. The related information includes identification information (for example, a serial number) or the like assigned in order to individually identify the capsule endoscope 2.

The receiving device 3 receives the image data and the related information wirelessly transmitted from the capsule endoscope 2 via the receiving antenna unit 4 including a plurality of receiving antennas 4a to 4h (eight receiving antennas in FIG. 1). Each of the receiving antennas 4a to 4h is realized by using a loop antenna for example, and arranged at a predetermined position (for example, a position corresponding to one of organs as a passage route of the capsule endoscope 2 in the subject 10) on an outside surface of a body of the subject 10.

As illustrated in FIG. 2, the receiving device 3 includes a receiving unit 31, a signal processing unit 32, a memory 33, a data transmitting unit 34, an operating unit 35, a display unit 36; a control unit 37, and a battery 38.

The receiving unit 31 receives the image data wirelessly transmitted from the capsule endoscope 2 via the receiving antennas 4a to 4h.

The signal processing unit 32 performs predetermined signal processing on the image data received by the receiving unit 31.

The memory 33 stores therein the image data subjected to the signal processing by the signal processing unit 32 and related information.

The data transmitting unit 34 is an interface connectable to a USB or a communication line, such as a wired LAN or a wireless LAN, and transmits the image data and the related information stored in the memory 33 to the image processing apparatus 5 under control of the control unit 37.

The operating unit 35 is used by a user to input various setting information or the like.

The display unit 36 display registration information on an examination (examination information, patient information, or the like), various setting information input by the user, or the like.

The control unit 37 controls operations of each unit in the receiving device 3.

The battery 38 supplies electric power to each unit in the receiving device 3.

The receiving device 3 is connected to the receiving antenna unit 4 attached to the subject 10 and is carried by the subject 10 while the capsule endoscope 2 is capturing images (a predetermined time after the capsule endoscope 2 is swallowed). During this period, the receiving device 3 stores, in the memory 33, the image data received via the receiving antenna unit 4 together with related information such as receiving intensity information and receiving time information in each of the receiving antennas 4a to 4h. After the capsule endoscope 2 completes the imaging, the receiving device 3 is removed from the subject 10, is then connected to the image processing apparatus 5, and transfers the image data and the related information stored in the memory 33 to the image processing apparatus 5. In FIG. 1, a cradle 3a is connected to a USB port of the image processing apparatus 5, and by setting the receiving device 3 in the cradle 3a, the receiving device 3 is connected to the image processing apparatus 5.

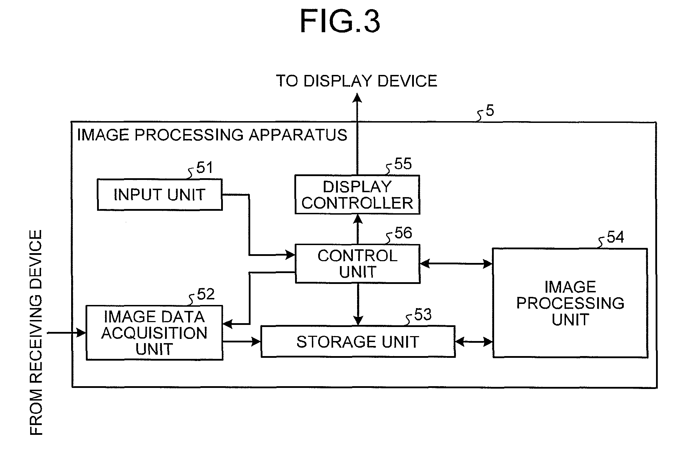

FIG. 3 is a block diagram illustrating a schematic configuration of the image processing apparatus 5. The image processing apparatus 5 is configured by using, for example, a workstation including a display device 5a, such as a CRT display or a liquid crystal display, and includes, as illustrated in FIG. 3, an input unit 51, an image data acquisition unit 52, a storage unit 53, an image processing unit 54, a display controller 55, and a control unit 56.

The input unit 51 is realized by an input device, such as a keyboard, a mouse, a touch panel, or various switches, and receives input of information and an instruction according to a user operation.

The image data acquisition unit 52 is an interface connectable to a USB or a communication line, such as a wired LAN or a wireless LAN, and includes a USB port, a LAN port, or the like. The image data acquisition unit 52 acquires the image data and the related information from the receiving device 3 via an external device such as the cradle 3a connected to the USB port or via various communication lines.

The storage unit 53 is realized by a semiconductor memory such as a flash memory, a RAM, a ROM, or a recording medium, such as an HDD, an MO, a CD-R, or a DVD-R, and a read and write device or the like that reads and writes information from and to the recording medium. The storage unit 53 stores therein programs and various information for causing the image processing apparatus 5 to operate and execute various function, image data acquired by capsule endoscopic examinations, or the like.

The image processing unit 54 is realized by hardware, such as a CPU, and by reading a predetermined program stored in the storage unit 53, performs predetermined image processing on image data acquired via the image data acquisition unit 52, generates an in-vivo image, and performs a process of generating an observation screen that contains the in-vivo image and that is in a predetermined format.

More specifically, the image processing unit 54 performs image processing for image generation (image processing for image generation to change to a format so that the stored image data can be displayed as an image), such as a white balance process, demosaicing, color conversion, density conversion (gamma conversion or the like), smoothing (noise elimination or the like), or sharpening (edge enhancement or the like), on the image data stored in the storage unit 53, and further performs image processing, such as a position detection process, an average color calculation process, a lesion detection process, a red detection process, an organ detection process, a predetermined feature detection process, on the generated images depending on purposes.

The display controller 55 causes the display device 5a to display, in a predetermined format, the in-vivo image generated by the image processing unit 54.

The control unit 56 is realized by hardware, such as a CPU, and by reading various programs stored in the storage unit 53, transfers instructions or data to each unit of the image processing apparatus 5 based on signals input via the input unit 51, image data acquired via the image data acquisition unit 52, or the like, and integrally controls the entire operations of the image processing apparatus 5.

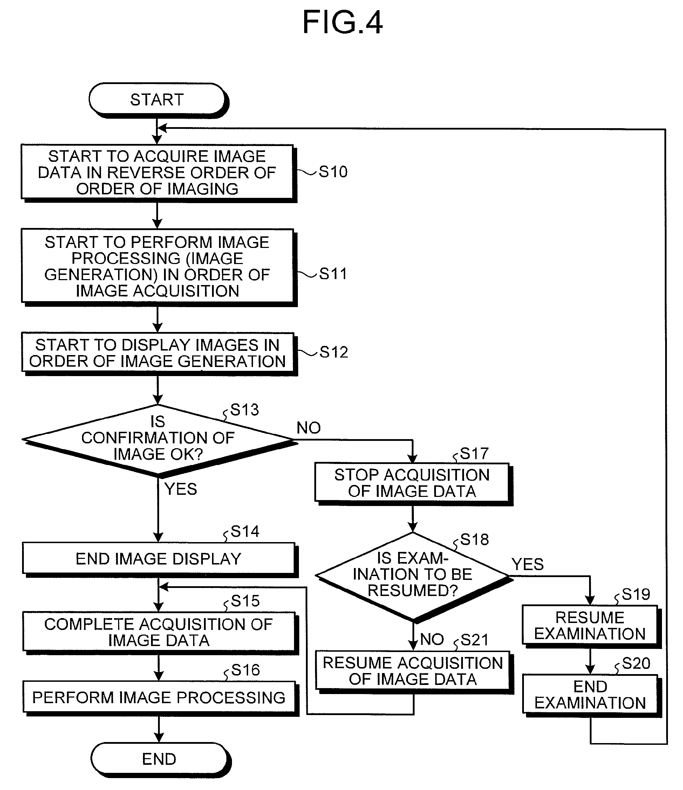

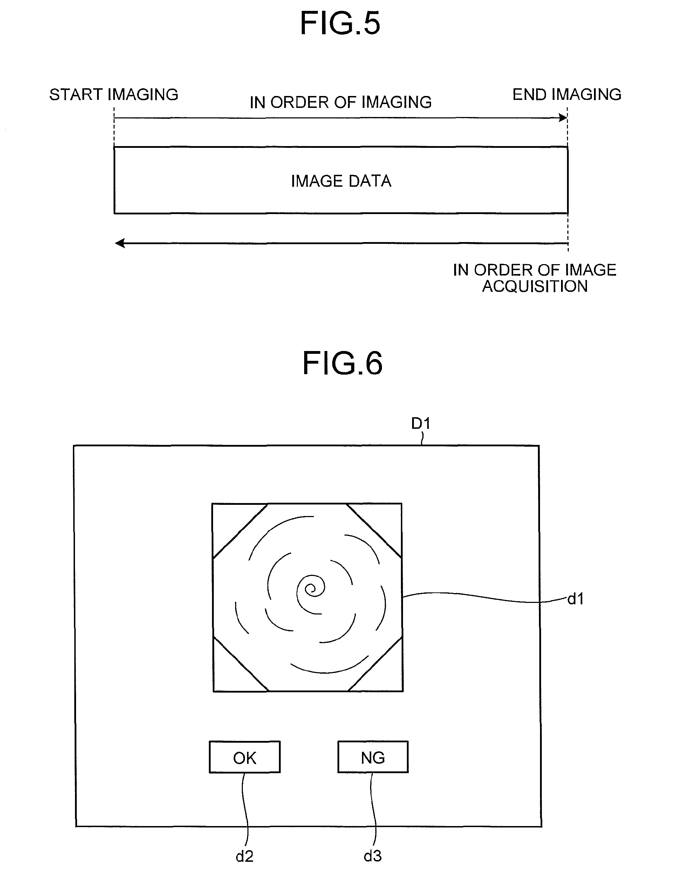

Next, operations of the image processing apparatus 5 will be described. FIG. 4 is a flowchart illustrating operations of the capsule endoscopic system 1 including the image processing apparatus 5. FIG. 5 is a schematic diagram for explaining operations of the image processing apparatus 5.

An examination using the capsule endoscope 2 is started when the subject 10 swallows the capsule endoscope 2 in a state in which the receiving antenna unit 4 is attached to the subject 10 and the receiving antenna unit 4 is connected to the receiving device 3. Thereafter, when a predetermined time has elapsed, the user (a medical worker) removes the receiving device 3 from the receiving antenna unit 4 and sets the receiving device 3 in the cradle 3a. The above described predetermined time is set to a time (for example, about 8 hours) enough for the capsule endoscope 2 to move inside the subject 10 by peristaltic movement and pass through an examination target region such as a small intestine. Furthermore, the user asks the subject 10 to wait because whether the examination needs to be resumed is uncertain at this stage.

In response to this, at Step S10, the image data acquisition unit 52 starts to acquire a series of image data accumulated in the receiving device 3. In this case, as illustrated in FIG. 5, the image data acquisition unit 52 acquires the image data in order from the latest imaging time, that is, in reverse order of the order in which the images are captured.

At subsequent Step S11, the image processing unit 54 starts to perform the image processing for image generation, such as a white balance process or demosaicing, on the image data acquired by the image data acquisition unit 52, in the order in which the image data are acquired. Even when the image processing unit 54 starts the image processing, the image data acquisition unit 52 continuously performs the process of acquiring image data in reverse order of the order in which the images are captured.

At Step S12, the display controller 55 displays images generated by the image processing unit 54 on the display device 5a in the order in which the images are generated. FIG. 6 is a schematic diagram illustrating a display example of a screen displayed on the display device 5a during acquisition of image data. A preview screen D1 illustrated in FIG. 6 is a screen for aiding a user to confirm whether an in-vivo image needed for a diagnosis of the subject 10 has been obtained, and contains an image display area d1, an OK button d2, and an NG button d3. The image display area d1 is an area for displaying in-vivo images generated by the image processing unit 54 in reverse chronological order from the end of the imaging. The OK button d2 and the NG button d3 are provided for the user to input a result of confirmation by using the input unit 51 including a mouse or the like.

The user observes the in-vivo images displayed on the image display area d1, and determines whether an image needed for the diagnosis has been obtained. For example, when an examination target region is the entire small intestine, and if the images displayed on the image display area d1 start with a large intestine, it is determined that a necessary image of the entire small intestine has been obtained. In this case, the user performs a predetermined pointer operation (for example, a click operation) on the OK button d2 by using a mouse or the like. In response to this, a signal (OK signal) indicating that the image is confirmed is input to the control unit 56.

At Step S13, if the OK signal is input to the control unit 56 (Step S13: Yes), the control unit 56 causes the display controller 55 to end display of the preview screen D1 (Step S14). Thereafter, the image data acquisition unit 52 continues to acquire image data in the background. The image processing for image generation may be temporarily suspended, and may be resumed after all of image data are acquired.

When the image is confirmed, the user may allow the subject 10 to go home.

At Step S15, when acquisition of all of the image data is completed, the image processing unit 54 performs the image processing for image generation and image processing for an individual predetermined purpose on the acquired image data in the order in which the images are captured (Step S16). However, it is sufficient to perform only the image processing for an individual purpose on image data for which images have already been generated before the end of the image display (Step S14). As the image processing for an individual purpose, a process is performed which is set in advance from among a position detection process, an average color calculation process, a lesion detection process, a red detection process, an organ detection process, a predetermined feature detection process, and the like. The reason why the image processing at Step S16 is performed in the same order as the order in which the images are captured is that the image processing for an individual purpose includes a process, such as a position detection process or a similarity detection process, in which the order of images is important because information on adjacent images is used.

When all of the image data are acquired by the image processing apparatus 5, the user may remove the receiving device 3 from the cradle 3a, clear away the devices or the like, and finish his/her work.

Subsequently, upon completion of the image processing for image generation and the image processing for an individual purpose on all of the image data acquired from the receiving device 3, the operations of the image processing apparatus 5 end. Thereafter, the image processing apparatus 5 may further generate and display an observation screen containing an in-vivo image according to a signal that is input from the input unit 51 by a user operation.

In contrast, when determining that an image needed for the diagnosis has not been obtained through the observation of the preview screen D1 illustrated in FIG. 6, the user performs a predetermined pointer operation (for example, a click operation) on the NG button d3 by using a mouse or the like. The case in which the image needed for the diagnosis has not been obtained is, for example, a case in which images displayed on the image display area d1 start with a middle of a small intestine while the examination target region is set to the entire small intestine, or a case in which image quality is extremely low due to the influence of noise or the like. In response to the pointer operation on the NG button d3, a signal (NG signal) indicating that the image is not confirmed is input to the control unit 56.

At Step S13, if the NG signal is input to the control unit 56 (Step S13: No), the control unit 56 causes the image data acquisition unit 52 to stop acquisition of image data (Step S17).

At this time, if the capsule endoscope 2 seems to be inside the subject 10, the user is able to resume the examination.

If the examination is to be resumed (Step S18: Yes), and when the user reconnects the receiving device 3 to the receiving antenna unit 4 and attaches the receiving antenna unit 4 to the subject 10, the examination is resumed (Step S19). In response to this, the receiving device 3 receives image data wirelessly transmitted from the capsule endoscope 2 via the receiving antenna unit 4. After an adequate time has elapsed since the resumption of the examination, and when the receiving device 3 is removed from the receiving antenna unit 4 again, the examination ends (Step S20). Thereafter, when the receiving device 3 is set in the cradle 3a again, the process returns to Step S10.

In contrast, if the examination is not to be resumed (Step S18: No), the image processing apparatus 5 causes the image data acquisition unit 52 to resume acquisition of image data (Step S21).

As described above, according to the first embodiment, in-vivo images generated in reverse chronological order from the end of the imaging are displayed while image data are being transferred from the receiving device 3 to the image processing apparatus 5. Therefore, the user is able to determine the necessity of a reexamination on the subject 10 at an earlier stage after the examination. Consequently, it becomes possible to reduce a wait time of the subject 10. Furthermore, if by any chance a necessary image has not been obtained, it is possible to immediately resume the examination. Therefore, it becomes possible to reduce a burden, such as a reexamination on another day, on the subject 10. Furthermore, the user is able to clear away the receiving device 3 upon completion of transfer of image data, so that it becomes possible to improve the efficiency of works related to examinations.

### (First Modified Example)

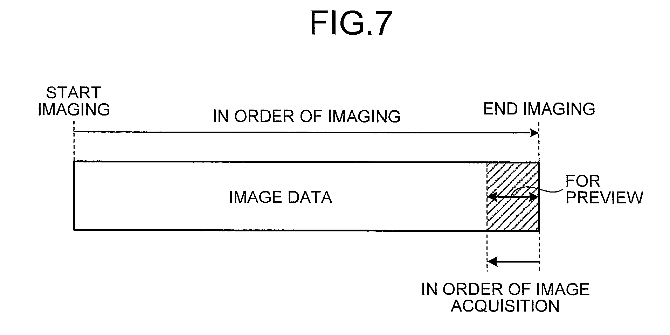

A first modified example of the first embodiment according to the present invention will be described. FIG. 7 is a schematic diagram for explaining operations of an image processing apparatus according to the first modified example.

In the above described first embodiment, the image processing apparatus 5 starts acquisition of image data accumulated in the receiving device 3 at the end of the imaging and continues the acquisition until the OK signal is input by a user operation on the preview screen D1. However, as indicated by diagonal lines in FIG. 7, it may be possible to acquire, as image data for preview, only image data generated in a predetermined period of time in reverse chronological order from the end of the imaging (namely, in a predetermined number of images).

In this case, if the user does not confirm images (if the OK signal or the NG signal is not input) even after acquisition of the preview image data is completed, the image processing apparatus 5 waits for input of the OK signal or the NG signal while displaying, on the display device 5a, a still image of the last in-vivo image generated for preview.

Furthermore, if the user confirms the images and the display of the preview screen is ended (see Step S14), it is preferable to acquire image data from the receiving device 3 to the image processing apparatus 5 in the order in which the images are captured, starting from the start of the imaging to the end of the imaging. As described above, by setting the order of acquisition of remaining image data to the same as the order of image processing at Step S16, it becomes possible to start image processing before the acquisition of the image data is completed (see Step S15), enabling to perform the acquisition of the image data and the image processing in parallel.

### (Second Embodiment)

A second embodiment of the present invention will be described.

A feature of an image processing apparatus according to the second embodiment lies in that it automatically determines whether a series of in-vivo images captured by the capsule endoscope 2 contains an in-vivo image needed for a diagnosis. A configuration of the image processing apparatus according to the second embodiment is the same as that of the image processing apparatus 5 illustrated in FIG. 3. Furthermore, a configuration of an entire capsule endoscopic system including the image processing apparatus is the same as illustrated in FIG. 1.

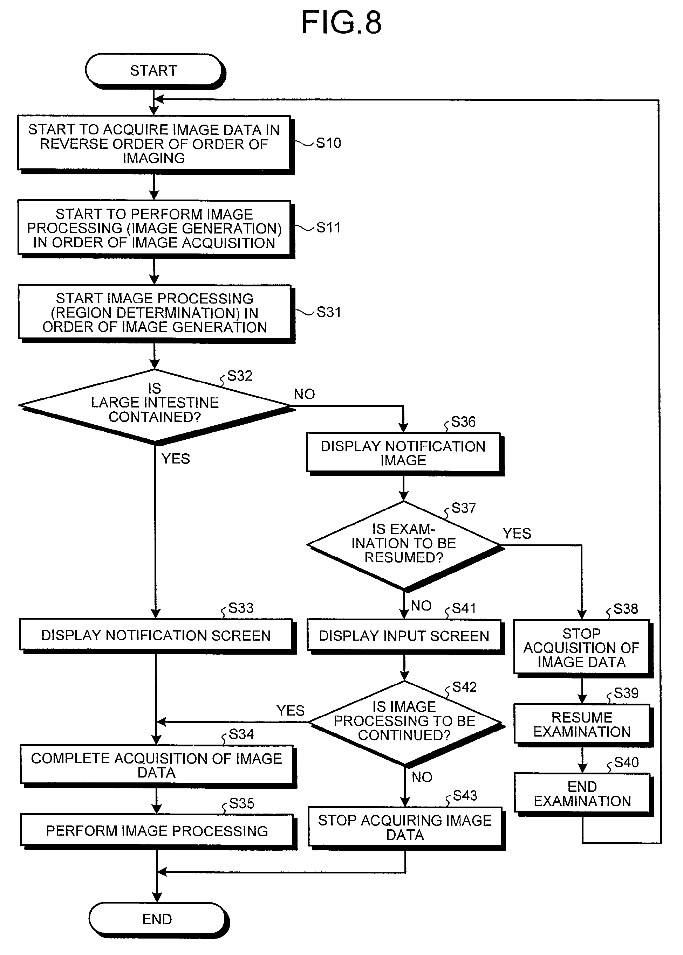

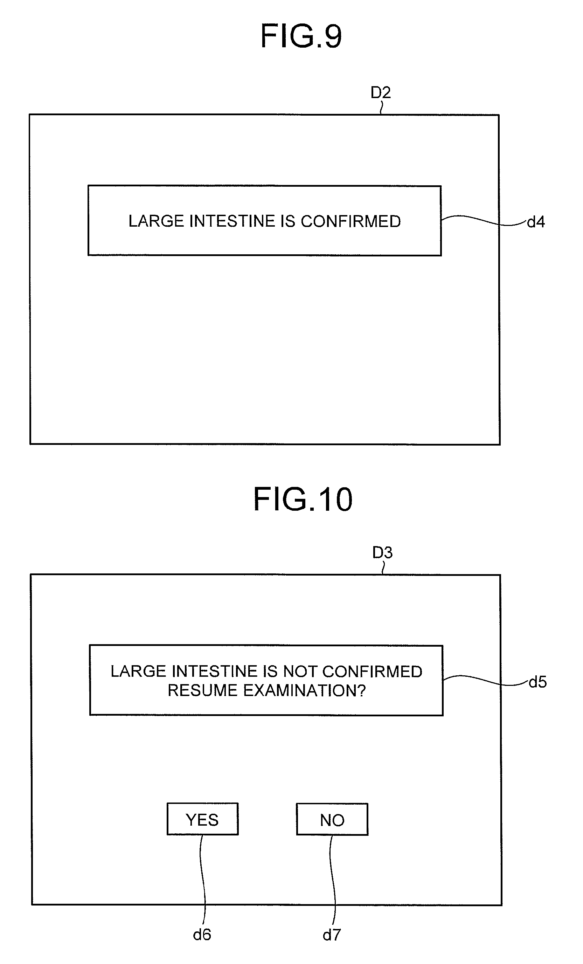

Operations of the image processing apparatus according to the second embodiment will be described. FIG. 8 is a flowchart illustrating operations of the capsule endoscopic system 1 including the image processing apparatus 5 according to the second embodiment. FIG. 9 to FIG. 11 are schematic diagrams illustrating screens displayed on the display device 5a. Steps S10 and S11 illustrated in FIG. 8 are the same as those of the first embodiment.

At Step S31 subsequent to Step S11, the image processing unit 54 starts to perform image processing of determining regions that appear in in-vivo images (a region determination process) on the image data that have been subjected to the image processing for image generation, in the order in which the images are generated (namely, the reverse order of the order in which the images are captured). As the region determination process, any well-known method may be used. For example, it may be possible to determine that, based on color feature data of the in-vivo images, a brownish in-vivo image corresponds to a large intestine and a yellowish in-vivo image corresponds to a small intestine.

As a result of the region determination by the image processing unit 54, if a large intestine is contained in the in-vivo images that are generated in reverse chronological order from the end of the imaging (Step S32: Yes), the display controller 55 displays, on the display device 5a, a screen for notifying that the large intestine is confirmed (Step S33). FIG. 9 is a schematic diagram illustrating a display example of a notification screen. In a message display field d4 provided in a notification screen D2 illustrated in FIG. 9, a text message "large intestine is confirmed" is displayed. The user may allow the subject 10 to go home after he/she has checked this display.

The notification to the user may be made not by the display of a text message but by, for example, a notification sound, a voice message, or the like. Alternatively, it may be possible to display an in-vivo image in which a region is confirmed, instead of or together with the display of a text message.

Thereafter, the image data acquisition unit 52 continues to acquire image data in the background. In this case, the image data may continuously be acquired in reverse order of the order in which the images are captured, or in the same order as the order in which the images are captured (namely, the same order as the subsequent image processing) similarly to the first modified example. Furthermore, the image processing for image generation may be temporarily suspended, and may be resumed after all of the image data are acquired.

At Step S34, when acquisition of all of the image data is completed, the image processing unit 54 performs the image processing for image generation and the image processing for an individual predetermined purpose on the acquired image data in the order in which the images are captured (Step S35). Incidentally, if the order of acquisition of the image data is changed to the order in which the images are captured, it may be possible to start the image processing before the acquisition of the image data is completed. Furthermore, it is sufficient to perform only necessary image processing on image data that have been subjected to the region determination process (Step S31).

When all of the image data are acquired by the image processing apparatus 5, the user may remove the receiving device 3 from the cradle 3a, clear away the devices or the like, and finish his/her work.

Thereafter, upon completion of the image processing for image generation and the image processing for an individual purpose on all of the image data acquired from the receiving device 3, the operations of the image processing apparatus 5 end. Incidentally, the image processing apparatus 5 may further generate and display an observation screen containing an in-vivo image according to a signal that is input from the input unit 51 by a user operation.

In contrast, when an image containing the large intestine is not detected even by performing the region determination process on a preset predetermined number of images at Step S31 (Step S32: No), the display controller 55 displays, on the display device 5a, a screen for notifying that the large intestine is not confirmed (Step S36). FIG. 10 is a schematic diagram illustrating a display example of a notification screen. In a message display field d5 provided in a notification screen D3 illustrated in FIG. 10, a text message "large intestine is not confirmed, resume examination?" is displayed. In this case, it may be possible to display in-vivo images subjected to the region determination, instead of or together with the text message. In this case, the user is able to confirm regions in the in-vivo images.

Furthermore, the notification screen D3 contains a YES button d6 and a NO button d7 to be used by the user to determine whether to resume the examination. When determining to resume the examination, the user performs a predetermined pointer operation (for example, a click operation) on the YES button d6 by using a mouse or the like. In response to this, a signal indicating that the examination is to be resumed is input to the control unit 56. In contrast, when determining not to resume the examination, the user performs a predetermined pointer operation on the NO button d7 by using a mouse or the like. In response to this, a signal indicating that the examination is not to be resumed is input to the control unit 56.

If the signal indicating that the examination is to be resumed is input (Step S37: Yes), the control unit 56 causes the image data acquisition unit 52 to stop acquisition of image data from the receiving device 3 (Step S38).

When the user reconnects the receiving device 3 to the receiving antenna unit 4 and attaches the receiving antenna unit 4 to the subject 10, the examination is resumed (Step S39). In response to this, the receiving device 3 receives image data wirelessly transmitted from the capsule endoscope 2 via the receiving antenna unit 4. After an adequate time has elapsed since the resumption of the examination, and when the the receiving device 3 is removed from the receiving antenna unit 4 again, the examination ends (Step S40). Thereafter, when the receiving device 3 is set to the cradle 3a again, the process returns to Step S10.

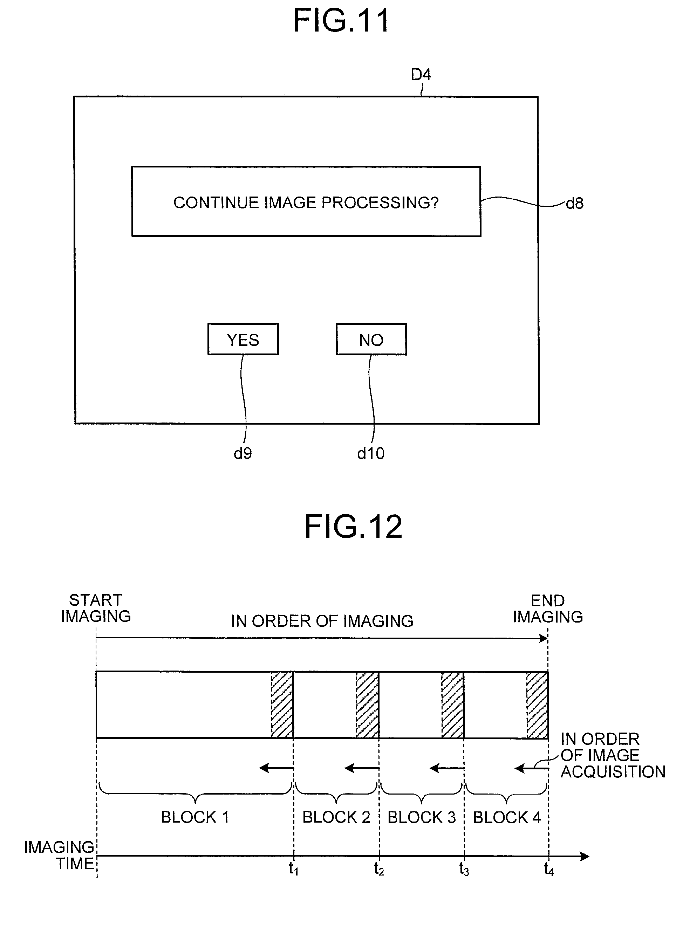

In contrast, if the signal indicating that the examination is not to be resumed is input to the control unit 56 (Step S37: No), the display controller 55 displays, on the display device 5a, an input screen for aiding the user to input an instruction on whether to cause the image processing apparatus 5 to continue image processing on image data that have already been accumulated in the receiving device 3 (Step S41). FIG. 11 is a schematic diagram illustrating a display example of the input screen. In a message display field d8 provided in an input screen D4 illustrated in FIG. 11, a text message "continue image processing?" is displayed. The user checks the display and determines whether to cause the image processing apparatus 5 to continue the image processing.

Furthermore, the input screen D4 contains a YES button d9 and a NO button d10 to be used by the user to determine whether to cause the image processing apparatus 5 to continue the image processing. When determining to continue the image processing, the user performs a predetermined pointer operation (for example, a click operation) on the YES button d9 by using a mouse or the like. In response to this, an instruction signal indicating that the image processing is to be continued is input to the control unit 56. In contrast, when determining not to continue the image processing, the user performs a predetermined pointer on the NO button d10 by using a mouse or the like. In response to this, an instruction signal indicating that the image processing is not to be continued is input to the control unit 56.

If the instruction signal indicating that the image processing is to be continued is input to the control unit 56 (Step S42: Yes), the operation of the image processing apparatus 5 proceeds to Step S34. In this case, the image processing apparatus 5 continues to acquire image data accumulated in the receiving device 3, and subsequently performs the image processing.

In contrast, if the instruction signal indicating that the image processing is not to be continued is input to the control unit 56 (Step S42: No), the control unit 56 causes the image data acquisition unit 52 to stop acquisition of image data from the receiving device 3 (Step S43).

As described above, according to the second embodiment, the region determination process is performs on in-vivo images generated in reverse chronological order from the end of the imaging while image data are being transferred from the receiving device 3 to the image processing apparatus 5. Therefore, the user is able to easily determine, at an earlier stage, whether the examination on the subject 10 needs to be resumed. Furthermore, the user is also able to determine, by himself/herself, whether to continue the image processing according to contents of individual examinations.

### (Second Modified Example)

A second modified example of the second embodiment according to the present invention will be described.

If the capsule endoscope 2 continues imaging after being excreted from the subject 10, an image obtained at the end of the imaging contains outside of the subject 10. Therefore, if the region determination process is performed on all of the images generated in reverse chronological order from the end of the imaging, it takes a longer time to reach images of inside of the subject 10.

Therefore, when the region determination process is performed at Step S31 in FIG. 8, it may be possible to skip images in which objects other than organs appear and exclude theses images from targets of the region determination process. The images to be skipped may be determined based on, for example, average colors of the images. In this case, the speed of the region determination process increases, so that it becomes possible to reduce a time to display the notification screen (see FIG. 9 and FIG. 10) for the user, in other words, a wait time of the user and the subject 10.

Furthermore, it may be possible to omit the region determination process on images that can obviously not be used for a diagnosis, such as images in which objects can hardly be distinguished due to halation, in addition to the images of outside of the subject 10. The halation images can be determined based on, for example, average luminance values of the images or the like.

### (Third Embodiment)

A third embodiment of the present invention will be described below.

A feature of an image processing apparatus according to the third embodiment lies in that a series of image data accumulated in the receiving device 3 are divided into a plurality of blocks, image data are acquired from each of the blocks, and image processing is performed on the acquired image data. A configuration of the image processing apparatus according to the third embodiment is the same as that of the image processing apparatus 5 illustrated in FIG. 3. Furthermore, a configuration and operations of an entire capsule endoscopic system including the image processing apparatus are the same as those illustrated in FIG. 1 and FIG. 4.

If, as described above, the capsule endoscope 2 continues imaging after being excreted from the subject 10, an image obtained at the end of the imaging contains outside of the subject 10. Therefore, if all of images generated in reverse chronological order from the end of the imaging are sequentially displayed on the preview screen, it takes a longer time to reach images of inside of the subject 10. Furthermore, in some cases, the user may want to confirm whether an image of a specific region inside the subject 10 has been obtained or whether there is a region whose image has not been obtained due to a failure in wireless transmission of image data caused by a failure of an antenna or the like.

Therefore, in the third embodiment, to enable the user to roughly grasp the entire series of in-vivo images obtained by an examination, the series of image data accumulated in the receiving device 3 is divided into a plurality of blocks, and images of a plurality of portions inside the subject 10 are simultaneously displayed as a preview.

More specifically, at Step S10 in FIG. 4, the image data acquisition unit 52 acquires, from a plurality of blocks 1 to 4 into which the series of the image data are divided, image data in reverse order of the order in which the images are captured, starting from the last imaging times t₁, t₂, t₃, and t₄ of the respective blocks as illustrated in FIG. 12. In this case, similarly to the first modified example, it may be possible to acquire, as image data for a preview screen, only image data generated in a predetermined period of time in reverse chronological order from the last imaging times t₁, t₂, t₃, and t₄ (namely, in a predetermined number of images) as indicated by diagonal lines in FIG. 12.

Furthermore, at subsequent Step S11, the image processing unit 54 performs the image processing for image generation on the image data acquired from each of the blocks 1 to 4 by the image data acquisition unit 52, in the order of in which the image data are acquired.

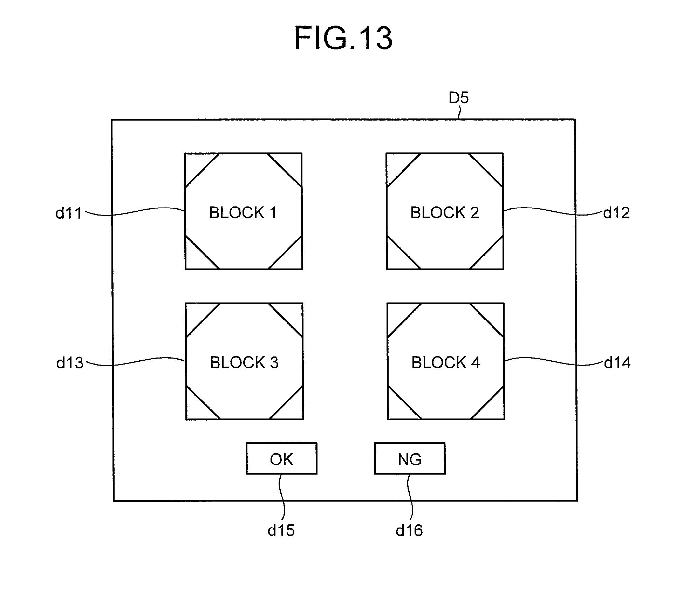

FIG. 13 is a schematic diagram illustrating an example of a screen displayed on the display device 5a during acquisition of image data. A preview screen D5 illustrated in FIG. 13 contains four image display areas d11 to d14 for respectively displaying image data acquired from the blocks 1 to 4, an OK button d15, and an NG button d16. The OK button d15 and the NG button d16 are used by the user who has observed the preview screen D5 to input a result of confirmation on whether an image needed for a diagnosis has been obtained, similarly to the OK button d2 and the NG button d3 illustrated in FIG. 6.

Incidentally, the image data may be transferred serially or in parallel from each of the blocks 1 to 4. If the image data are transferred serially, the image data acquisition unit 52 moves between the blocks in order of, for example, the block 4 → the block 3 → the block 2 → the block 1 → the block 4 → ..., and acquires a predetermined amount of image data (for example, one image for each). In this case, in the preview screen D5, in-vivo images displayed on the image display areas d11 to d14 are switched one by one in reverse chronological order of the imaging time.

Furthermore, if the image data are transferred in parallel, the image data acquisition unit 52 simultaneously acquires predetermined amounts of image data from the blocks 1 to 4. In this case, the image processing unit 54 performs, in parallel, the image processing on the image data acquired from the respective blocks 1 to 4. Furthermore, in the preview screen D5, in-vivo images displayed on the image display areas d11 to d14 are simultaneously switched in in reverse chronological order of the imaging time.

As described above, according to the third embodiment, the user is able to roughly grasp the entire series of in-vivo images obtained by an examination. Therefore, it becomes possible to easily and accurately determine whether an image needed for a diagnosis has been obtained.

In the above described third embodiment, it may be possible to perform the region determination process (see Step S31 in FIG. 8), instead of displaying a preview of the images acquired from the blocks 1 to 4. In this case, it is preferable to provide areas for displaying results of the region determination near the image display areas d11 to d14 illustrated in FIG. 13.

The above described present invention is not limited to the first to third embodiments and the modified examples thereof, and various inventions may be formed by appropriately combining a plurality of structural elements disclosed in the respective embodiments and modified examples. For example, formation by excluding some of the structural elements from the whole structural elements illustrated in the respective embodiments and modified examples may be made, or formation by appropriately combining the structural elements illustrated in the different embodiments and modified examples may be made.

### Reference Signs List

- 1: CAPSULE ENDOSCOPIC SYSTEM
- 2: CAPSULE ENDOSCOPE
- 3: RECEIVING DEVICE
- 3a: CRADLE
- 4: RECEIVING ANTENNA UNIT
- 4a: TO 4h RECEIVING ANTENNA
- 5: IMAGE PROCESSING APPARATUS
- 5a: DISPLAY DEVICE
- 10: SUBJECT
- 21: IMAGING UNIT
- 22: ILLUMINATION UNIT
- 23: SIGNAL PROCESSING UNIT
- 24, 33: MEMORY
- 25: TRANSMITTING UNIT
- 26: ANTENNA
- 27, 38: BATTERY
- 31: RECEIVING UNIT
- 32: SIGNAL PROCESSING UNIT
- 34: DATA TRANSMITTING UNIT
- 35: OPERATING UNIT
- 36: DISPLAY UNIT
- 37: CONTROL UNIT
- 51: INPUT UNIT
- 52: IMAGE DATA ACQUISITION UNIT
- 53: STORAGE UNIT
- 54: IMAGE PROCESSING UNIT
- 55: DISPLAY CONTROLLER
- 56: CONTROL UNIT

## Claims

1. An image processing apparatus that processes, in a capsule endoscopic system including a capsule endoscope introduced into a subject and wirelessly transmitting a series of image data generated by capturing images inside the subject, and a receiver receiving and accumulating the series of the image data wirelessly transmitted from the capsule endoscope, the image data acquired from the receiving device, the image processing apparatus comprising:
an image data acquisition unit that sequentially acquires the image data from the receiving device in order from a latest imaging time;
an image processing unit that performs predetermined image processing on the image data acquired by the image data acquisition unit, in order in which the image data are acquired; and
a display controller that displays a screen containing a result obtained through the predetermined image processing.

2. The image processing apparatus according to claim 1, wherein
the image processing unit generates images based on the image data, and
the display controller displays the images generated by the image processing unit in order in which the images are generated.

3. The image processing apparatus according to claim 1, wherein the image data acquisition unit acquires image data generated in a predetermined period of time in reverse chronological order from the latest imaging time of the capsule endoscope.

4. The image processing apparatus according to claim 1, wherein
the image processing unit generates images based on the image data, and determines whether or not a specific region inside the subject appears in the images,
the display controller displays a result of determination performed by the image processing unit.

5. The image processing apparatus according to claim 4, wherein the image processing unit determines whether an organ inside the subject appears in the images based on the image data, and subsequently determines, with respect to images in which the organ appears, whether or not the organ in the images is the specific region.

6. The image processing apparatus according to claim 1, wherein
the image data acquisition unit divides the series of image data captured in one examination by the capsule endoscope into a plurality of blocks, and acquires image data in order from a latest imaging time of each of the blocks,
the image processing unit sequentially generates images based on the image data, and
the display controller displays a screen containing a plurality of image display areas for displaying a plurality of images based on the image data acquired from the respective blocks.

7. The image processing apparatus according to claim 6, wherein the image data acquisition unit acquires a part of the image data from each of the blocks while sequentially changing the group from which the image data is acquired among the blocks.

8. The image processing apparatus according to claim 6, wherein the image data acquisition unit acquires the image data from the plurality of the blocks in parallel.

9. An image processing method of processing, in a capsule endoscopic system including a capsule endoscope introduced into a subject and wirelessly transmitting a series of image data generated by capturing images inside the subject, and a receiver receiving and accumulating the series of the image data wirelessly transmitted from the capsule endoscope, the image data acquired from the receiving device, the image processing method comprising:
an image data acquiring step of acquiring the image data sequentially from the receiving device in order from a latest imaging time;
an image processing step of performing predetermined image processing on the image data acquired at the acquiring, in order in which the image data are acquired; and
a display controlling step of displaying a screen containing a result obtained through the predetermined image processing.
